# EUROPEAN PATENT APPLICATION

(11) **EP 1 721 570 A1**
(43) Date of publication of application: **15.11.2006**
(21) Application number: 06250195.2
(22) Date of filing: 13.01.2006
(51) Int. Cl.: A61B 5/00, G01G 19/414

(54) **Weight management system**

(30) Priority: 12.05.2005 US 127191
(71) Applicant: IDT Technology Limited, Kowloon, Hong Kong SAR (CN)
(72) Inventor: Chan, Raymond, Hunghom, Kowloon (HK)
(74) Representative: Jenkins, Richard Gavin

(57) **Abstract**

A weight management system (100) includes a control wristwatch (10) including a processor (9) programmed with a weight management program and an RF receiver, a weight scale (12) for collecting weight data from a user and including an RF transmitter for transmitting the weight data to the wristwatch (10), and a pedometer (14) or heart rate monitor (15) for collecting exercise data from the user and including an RF transmitter for transmitting the exercise data to the wristwatch (10). The weight management program is operable on such weight data to determine a plan for managing the weight of the user through exercise and on such exercise data to monitor execution of the plan.

## Description

The present invention relates to a weight management system or apparatus.

### BACKGROUND OF THE INVENTION

There is a disturbing trend in many countries towards popular obesity, especially in children. Diminished life expectancy and general poor health amongst the obese are well documented.

Numerous fad diets, gadgets and other time-wasting proposals have been put forward over the years. Some useful devices including pedometers, heart rate monitors, blood pressure monitors and the like encourage people to exercise more. Some devices calculate calorific expenditure, whereas others store a weight record in electronic memory. These known systems suffer from the requirement for regular direct input of data by the user including body weight and calorific expenditure for example. This can be inconvenient and burdensome and is often forgotten.

### OBJECT OF THE INVENTION

It is an object of the present invention to overcome or substantially ameliorate the above disadvantages and/or more generally to provide an improved weight management system.

### SUMMARY OF THE INVENTION

According to the invention, there is provided a weight management system which comprises a control unit including a processor programmed with a weight management program, memory for data storage, and a wireless receiver, a weight data unit for collecting weight data from a user and including a wireless transmitter for transmitting said weight data to the control unit as received by the receiver, and an exercise data unit for collecting exercise data from said user and including a wireless transmitter for transmitting said exercise data to the control unit as received by the receiver. The weight management program is operable on said weight data to determine a plan for managing the weight of said user through exercise and on said exercise data to monitor execution of the plan.

Preferably, the memory stores exercise calorie data for use by the processor in determining the plan, the exercise calorie data being indicative of amount in calories for expending through exercise by users of different weight classifications.

In a preferred embodiment, the processor is programmed to identify a target weight data and based on said target weight data to recommend a level of exercise comprised in the plan.

More preferably, the control unit includes a transmitter for transmitting said level of exercise to the exercise data unit.

More preferably, the target weight data comprises a reduction in weight over a period of time.

More preferably, the target weight data is selectable by said user to adjust the plan.

More preferably, the processor is programmed to check at the end of a predetermined period whether a target represented by the target weight data is met, and to permit adjustment of the plan by said user for further operation if the target is not met.

In a preferred embodiment, the plan includes recommendation on calorie intake by said user.

It is preferred that the control unit includes means for requesting operation of the weight data unit by said user to collect an update of weight data from said user.

Preferably, the control unit is in the form of a wristwatch.

Preferably, the weight data unit comprises a weight scale.

Preferably, the exercise data unit comprises a pedometer.

Preferably, the exercise data unit comprises a heart rate monitor.

### BRIEF DESCRIPTION OF DRAWINGS

The invention will now be more particularly described, by way of example only, with reference to the accompanying drawings, in which:
Figure 1 is a schematic diagram of an embodiment of a weight management system in accordance with the invention, depicting all major hardware components thereof; and
Figure 2 is an operation flowchart illustrating a weight management program for execution using the system of Figure 1.

### DETAILED DESCRIPTION OF PREFERRED EMBODIMENT

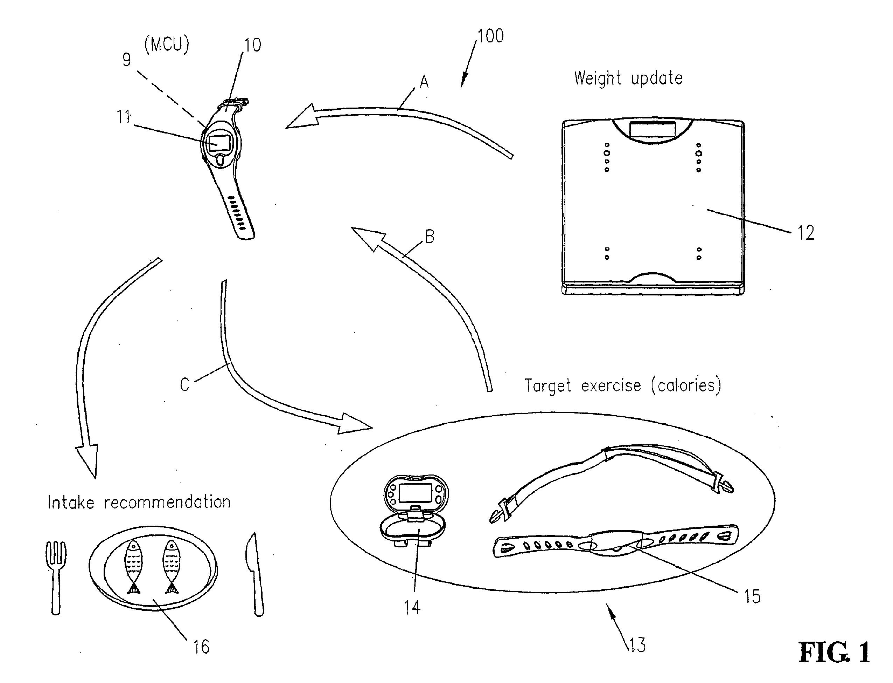

Referring initially to Figure 1 of the drawings, there are shown various hardware components of a weight management system 100 embodying the invention, which includes a control unit 10, a weight data unit 12 and an exercise data unit 13. The control unit 10 and the weight/exercise data unit 12/13 are adapted to communicate with each other via RF (radio frequency) in a wireless manner.

The control unit 10 is preferably wrist-mountable and conveniently takes the form of a wristwatch 10 which has a display 11 and includes a number of input keys, an internal control circuit incorporating a processor 9 with memory for operation and control and a wireless RF transceiver (transmitter/receiver). The processor 9 is preferably an MCU i.e. a microprocessor control unit or microcomputer unit.

The weight data unit 12 is preferably implemented in or provided by a weight scale 12, which apart from the weighing mechanism includes a wireless RF transmitter to transmit weight measurement data for reception by the RF transceiver of the wristwatch 10 as shown by arrow A.

The exercise data unit 13 preferably comprises a pedometer 14 and (or) a chest belt-mounted heart rate monitor 15, each of which includes a wireless RF transceiver to transmit calorific consumption data for reception by the RF transceiver of the wristwatch 10 as shown by arrow B.

In operation, the user should initially use the input keys of the wristwatch 10 to enter certain personal data including gender, age, body height and present weight for processing by the MCU of the wristwatch 10. These data are stored in a user record, acting as a basis for managing the weight of this specific user according to a weight management program.

To commence the weight management program, the user must also enter or select a target weight data. The wristwatch 10 is preferably programmed to reject an unreasonably low (i.e. unachievable) target weight should this be entered. Once the program commences, the wristwatch 10 sounds an alarm and/or displays a message to requesting a weight update. This requires the user to step on the weight scale 12, and the measured weight is then transmitted by RF communication to the wristwatch 10 for updating the user's record.

The MCU of the wristwatch 10 will then determine an exercise plan and a calorie intake plan and display the same for direct readout at the wristwatch display 11 as recommendation to the user. The exercise plan specifies the minimum amount of energy measured in calories that should be burnt by exercise daily or every week, i.e. level of exercise. The calorie intake plan is a dietary plan that indicates the maximum amount of food/drink expressed in calories that should be taken every day. The user should follow these two plans as closely as possible in doing exercise and taking food and drink.

The suggested level of exercise may at the same time be transmitted to the pedometer 14 and the heart rate monitor 15 as shown by arrow C, for example in proportion especially when different types of exercise are recommended.

At the completion of each session of exercise, the pedometer 14 or heart rate monitor 15 will automatically as preferred, or upon pressing of a SEND key thereon if appropriate, collect and transmit the relevant body-sensed exercise data wirelessly to the wristwatch 10 as a feedback for data update to monitor execution of the exercise. The user should regularly check his/her weight using the scale 12, which in response will automatically as preferred, or upon pressing of a SEND key thereon if appropriate, collect and transmit the weight data wirelessly to the wristwatch 10 for data update to keep a weight history for the user.

The weight management program specifies a target reduction of the user's weight over a certain period of time, such as one or two weeks or up to say one month. At the end of this period, the wristwatch 10 will review the weight history and the exercise feedbacks and carry out the appropriate computation and comparison to check whether or not the target weight (or loss in weight) is achieved. In the negative (i.e. the aforesaid two plans fails), the wristwatch 10 will suggest a relatively heavier exercise plan (i.e. do more exercise) and/or a relatively lighter calorie intake plan (i.e. take less food/drink).

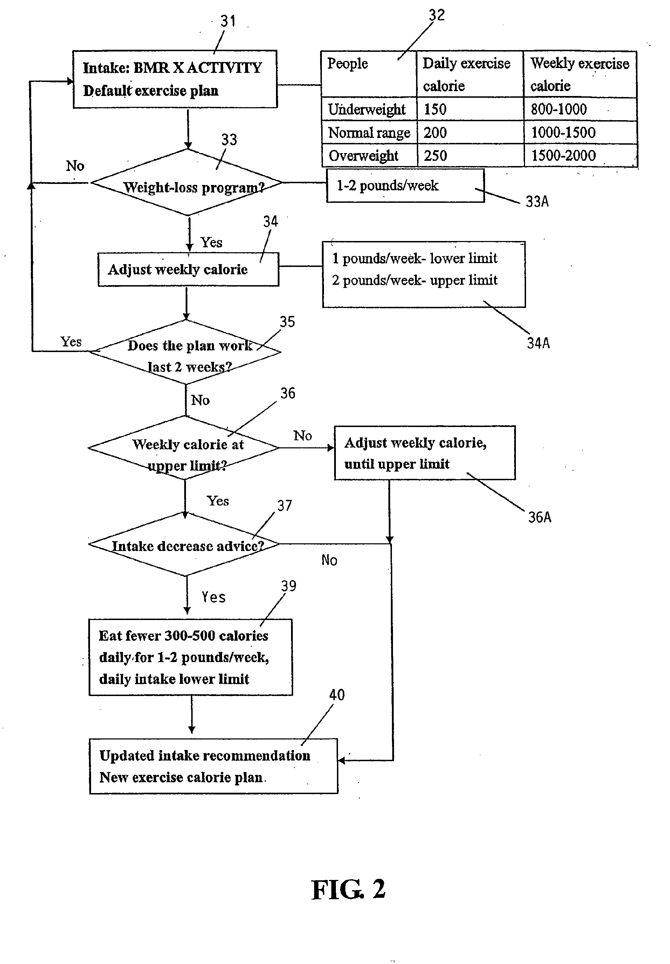

Figure 2 illustrates the major functions of the weight management program of the subject system 100 as performed by the MCU, which is programmed to and includes means to perform the various functions. In particular, the way the exercise and calorie intake plans are determined by the wristwatch 10 and adjusted by users is demonstrated.

The calorie intake plan is determined based on the Basal Metabolic Rate (BMR) in combination with the exercise activity level (step 31). BMR is the minimum calorific requirement needed to sustain life in a resting body, or the amount of energy in calories expended by the body to remain in bed asleep all day. BMR can be responsible for burning up to 70% of the total calories expended, with the remainder being burned by activities of the person and this can considerably be enhanced by doing exercises.

The default exercise plan (box 31) is based on an exercise calorie chart 32, which classifies the weight of people into three groups, namely underweight, normal range and overweight. Each group is assigned with daily and weekly exercise calorie expenditure values, e.g. an overweight person should burn 1500-2000 calories per week through exercise.

A target weight should first be entered or selected to commence a weight-loss program (box 33) as mentioned above, which is expressed as, for example, a loss in weight of 1-2 pounds per week (box 33A) . According to the exercise calorie chart 32, an overweight user should expend at least 1500 calories (lower limit) to lose 1. pound per week or at least 2000 calories (upper limit) to lose 2 pounds per week (box 34A). The rate of weight loss can be selected by the user, using the keys on the wristwatch 10, to adjust the weekly calorie burning of the exercise plan (box 34).

Once the target weight loss or exercise plan is determined, the MCU of the wristwatch 10 will recommend a corresponding calorie intake plan, dependent also upon other factors such as the gender, age and body height. The weight management program then commences, and self-discipline on exercises and dietary control is required.

The wristwatch 10 will provide one or more weight update alarms, for example weekly, to ask the user to measure his/her weight using the weight scale 12 for updating the weight history in the internal memory. The wristwatch 10 will also receive feedback of exercise data from the pedometer 14 or heart rate monitor 15 after each session of exercise.

According to the weight management program, the weight loss progress is checked at the end of, say, two weeks from commencement (box 35). If the target weight loss is met, the exercise and calories intake plans work and the program should continue for another two weeks until the result is examined again, and so on. If there is insufficient reduction in weight, the plans are not adequate and they will be reviewed.

The wristwatch 10 will first check whether the upper limit of the weekly exercise calorie has been set (box 36). In the negative, the wristwatch 10 will notify the user to adjust the calorie expenditure to or towards the upper limit (box 36A) and then propose a new, harder exercise plan (box 40). In the affirmative, the wristwatch 10 will enquire whether the user is willing to accept a calories intake decrease advice (box 37).

In case the user refuses, he/she may choose to retry the same exercise and calories intake plans for another two weeks (box 40). If the user agrees to a stricter dietary control, the wristwatch 10 will recommend daily intake reduction of say 300-500 calories for 1-2 pounds loss per week, above a predetermined daily intake lower limit (box 39), and update the calorie intake plan (box 40) for further operation of the program.

The weight management program as described above is interactive with the user, in that either of the exercise and calorie intake plans can be adjusted according to the progress of reduction in the user's weight as suggested by the program, subject to agreement of the user.

It should be appreciated that modifications of or alterations to the described embodiment obvious to those skilled in the art are not to be considered as beyond the scope of the present invention. For example, rather than using a separate weight scale, the weight data collector 12 might be built into the exercise apparatus to which the exercise data collector 13 is installed. As an alternative, the control unit 10 may be worn or clipped on a waist belt.

## Claims

1. A weight management system comprising:
a control unit including a processor programmed with a weight management program, memory for data storage, and a wireless receiver;
a weight data unit for collecting weight data from a user and including a wireless transmitter for transmitting said weight data to the control unit as received by the receiver; and
an exercise data unit for collecting exercise data from said user and including a wireless transmitter for transmitting said exercise data to the control unit as received by the receiver;
the weight management program being operable on said weight data to determine a plan for managing the weight of said user through exercise and on said exercise data to monitor execution of the plan.

2. The weight management system as claimed in claim 1, **characterized in that** the memory stores exercise calorie data for use by the processor in determining the plan, the exercise calorie data being indicative of amount in calories for expending through exercise by users of different weight classifications.

3. The weight management system as claimed in claim 1, **characterized in that** the processor is programmed to identify a target weight data and based on said target weight data to recommend a level of exercise comprised in the plan.

4. The weight management system as claimed in claim 3, **characterized in that** the control unit includes a transmitter for transmitting said level of exercise to the exercise data unit.

5. The weight management system as claimed in claim 3 or claim 4, **characterized in that** the target weight data comprises a reduction in weight over a period of time.

6. The weight management system as claimed in claim 3 or claim 4, **characterized in that** the target weight data is selectable by said user to adjust the plan.

7. The weight management system as claimed in claim 3 or claim 4, **characterized in that** the processor is programmed to check at the end of a predetermined period whether a target represented by the target weight data is met, and to permit adjustment of the plan by said user for further operation if the target is not met.

8. The weight management system as claimed in any one of claims 1 to 4, **characterized in that** the plan includes recommendation on calorie intake by said user.

9. The weight management system as claimed in any one of claims 1 to 4, **characterized in that** the control unit includes means for requesting operation of the weight data unit by said user to collect an update of weight data from said user.

10. The weight management system as claimed in any one of claims 1 to 4, **characterized in that** the control unit is in the form of a wristwatch.

11. The weight management system as claimed in any one of claims 1 to 4, **characterized in that** the weight data unit comprises a weight scale.

12. The weight management system as claimed in any one of claims 1 to 4, **characterized in that** the exercise data unit comprises a pedometer.

13. The weight management system as claimed in any one of claims 1 to 4, **characterized in that** the exercise data unit comprises a heart rate monitor.
